# EUROPÄISCHE PATENTSCHRIFT

(11) **EP 0 857 009 B1**
(45) Veröffentlichungstag und Bekanntmachung des Hinweises auf die Patenterteilung: **08.11.2006**
(21) Anmeldenummer: 98200132.3
(22) Anmeldetag: 20.01.1998
(51) Int. Cl.: H05G 1/36, H05G 1/44

(54) **Röntgeneinrichtung mit einer Primärblendenanordnung**
X-ray apparatus with a primary diaphragm
Appareil à rayons X comportant un diaphragme primaire

(30) Priorität: 27.01.1997 DE 19702739
(43) Veröffentlichungstag der Anmeldung: 05.08.1998
(73) Patentinhaber: Philips Intellectual Property & Standards GmbH, 20099 Hamburg (DE); Koninklijke Philips Electronics N.V., 5621 BA Eindhoven (NL)
(72) Erfinder: Brendler, Joachim, c/o Philips Patentverwaltung, 22335 Hamburg (DE); Allmendinger, Horst, c/o Philips Patentverwaltung, 22335 Hamburg (DE)
(74) Vertreter: Volmer, Georg

(56) Entgegenhaltungen:
- EP-A- 0 121 151
- EP-A- 0 480 096
- DE-A- 2 411 630
- DE-A- 3 006 774
- DE-A- 3 802 351
- DE-A- 4 411 729
- US-A- 4 573 183

## Beschreibung

Die Erfindung betrifft eine Röntgeneinrichtung mit nach dem Oberbegriff des Anspruchs 1. Eine derartige Röntgeneinrichtung ist aus der DE-A 30 06 774 bekannt. Der Regelkreis weist dabei einen Addierer auf, der einem Sollwert zwei Speicherwerte überlagert, wobei der erste Speicherwert die Abhängigkeit des Konversionsfaktors eines als Röntgenbildwandler verwendeten Röntgenbildverstärkers von der Elektrodenspannung und der zweite Speicherwert die Abhängigkeit des Untergrundes (der Resthelligkeit) des Röntgenbildverstärkers von der das Bildformat bestimmenden Öffnung der Primärblendenanordnung enthält. Dadurch wird eine Korrektur der durch den Röntgenbildverstärker verfälschten Meßwerte für die automatische Belichtungsregelung erreicht.

Aus der DE A 38 02 351 ist eine Röntgeneinrichtung bekannt, bei der der Dominantenbereich eines Detektors kontinuierlich einstellbar ist. Um ein von der Größe dieses Dominantenbereiches unabhängiges Ausgangssignal zu erzielen, wird die Verstärkung des Detektorsignals entsprechend geändert. - Schließlich ist aus der DE A 4411729 eine zahnärztliches Röntgendiagnostikgerät bekannt, das einen Strahlendetektor aufweist, mit dessen Hilfe das Strahlenfeld - wenn es außerhalb eines vorbestimmten Bereiches auf einen Strahlenempfänger trifft - auf den Strahlenempfänger ausrichtet.

Im Röntgenbild soll der Bereich, der von diagnostischem Interesse ist, eine bestimmte mittlere Schwärzung aufweisen. Das Meßfeld des Detektors wird mit diesem interessierenden Bereich zur Deckung gebracht, und in diesem Meßfeld wird während der Bildaufnahme laufend durch Erfassung der Röntgenstrahlendosisleistung die bereits aufgelaufene Röntgenstrahlendosis gemessen. Ist eine vorgegebene Röntgenstrahlendosis erreicht, die einem mittleren Schwärzungsgrad entspricht, bewirkt die automatische Belichtungsregelung eine Abschaltung der Röntgenstrahlung.

Um die Größe des vom Röntgenstrahlenbündel durchsetzten Bereiches den medizinischen Erfordernissen anzupassen und auch um ggf. die kontrastmindernde Wirkung von Streustrahlung zu reduzieren, ist zwischen der Röntgenstrahlenquelle und einem Untersuchungsobjekt, beispielsweise einem Patienten, eine Primärblendenanordnung zur Begrenzung (Einblendung) des Röntgenstrahlenbündels vorgesehen. Wird jedoch so stark eingeblendet, d.h., wird die Blendenöffnung so stark verkleinert, daß Teilbereiche des Meßfeldes von den Blenden der Primärblendenanordnung abgeschattet und nicht mehr belichtet werden, führt dies bei der herkömmlichen Belichtungsregelung dazu, daß überbelichtete Röntgenbilder entstehen. Dies liegt daran, daß der Detektor die Dosis im gesamten Meßfeld durch Mittelung erfaßt, auch wenn Teilbereiche des Meßfeldes durch die Blenden abgeschattet und unbelichtet sind. Die über den gesamten Bereich des Meßfeldes gemittelte Dosis ist deshalb bei starker Einblendung geringer als die im belichteten Bereich des Meßfeldes erreichte Dosis. Die herkömmliche automatische Belichtungsregelung führt dann zu einer Erhöhung der Röntgenstrahlendosis, bis die vorgegebene Röntgenstrahlendosis erreicht ist. Das Ergebnis sind oft überbelichtete Röntgenbilder, in denen wichtige Bildinformationen verloren gegangen sind, und eine erhöhte Dosisbelastung des Patienten.

Der Erfindung liegt deshalb die Aufgabe zugrunde, eine Röntgeneinrichtung der eingangs genannten Art zur Erzeugung von Röntgenbildern mit verbesserter Bildqualität anzugeben.

Diese Aufgabe wird ausgehend von einer eingangs genannten Röntgeneinrichtung dadurch gelöst, daß Mittel zur Bestimmung eines den Regelkreis beeinflussenden Korrekturwertes, der von der Größe des belichteten Bereiches des Meßfeldes abhängig ist, und zur Beeinflussung des Regelkreises vorgesehen sind derart, daß auch bei bereichsweise unbelichtetem Meßfeld die Röntgenstrahlendosis nahezu unverändert bleibt.

Dadurch wird unmittelbar bei der Regelung berücksichtigt, wenn Teilbereiche des Meßfeldes unbelichtet sind, weil die Größe der Öffnung der Primärblendenanordnung einen Grenzwert unterschreitet. In diesem Fall wird mittels des Korrekturwertes unmittelbar in den Regelkreis eingegriffen, um zu verhindern, daß überbelichtete Röntgenbilder entstehen, in denen wichtige Bildinformationen fehlen. Die Regelung erfolgt dabei derart, daß die Röntgenstrahlendosis bei kleiner Primärblendenöffnung (und teilweise unbelichtetem Meßfeld) nahezu konstant gehalten wird und insgesamt etwa genauso hoch ist wie bei großer Primärblendenöffnung (und vollständig belichtetem Meßfeld). Im Gegensatz dazu würde bei der herkömmlichen Regelung die Röntgenstrahlendosis bei kleiner Primärblendenöffnung deutlich erhöht werden. Durch die Erfindung wird also eine Erhöhung der Röntgenstrahlendosis bei starker Einblendung verhindert.

In einer Ausgestaltung der erfindungsgemäßen Röntgeneinrichtung ist vorgesehen, daß der Regelkreis Mittel zur Bestimmung eines Dosis-Istwertes, eines Dosis-Sollwertes und eines Steuersignals durch Vergleich des Dosis-Istwertes mit dem Dosis-Sollwert zur Steuerung der Röntgenstrahlenquelle aufweist. Erfindungsgemäß kann dieser Regelkreis mittels des Korrekturwertes derart beeinflußt werden, daß bei einer teilweisen Abschattung des Meßfeldes aufgrund einer kleinen Primärblendenöffnung der Dosis-Sollwert ausgehend von einem vorgegebenen Dosis-Standardsollwert verringert und/oder der Dosis-Istwert, der vom Detektor im Meßfeld erfaßt wird, erhöht wird. Die Dosis-Sollwerte können dabei in einem Speicher abgelegt und damit vorgegeben sein oder abhängig von verschiedenen Einstellungen der Röntgeneinrichtung wie dem Abstand zwischen der Röntgenstrahlenquelle und dem Röntgenbildwandler und dem Vergrößerungsfaktor des Röntgenbildwandlers ausgewählt oder bestimmt werden. Das berechnete Steuersignal dient dazu, die Röntgenstrahlenquelle bzw. einen Röntgengenerator zu steuern durch Beeinflussung von Röhrenstrom, Röhrenspannung und/oder Belichtungszeit.

In einer weiteren Ausgestaltung der erfindungsgemäßen Röntgeneinrichtung ist vorgesehen, daß die Mittel zur Bestimmung des Korrekturwertes eine Recheneinheit zur Bestimmung des Korrekturwertes durch Bildung des Verhältnisses aus der Fläche des belichteten Bereiches des Meßfeldes zur Gesamtfläche des Meßfeldes und daß der Regelkreis eine Multipliziereinheit zur Bestimmung des Dosis-Sollwertes durch Multiplikation eines vorgegebenen Dosis-Standardsollwertes mit dem Korrekturwert aufweisen. Dies stellt eine einfache Lösung zur Bestimmung des Korrekturwertes und des Dosis-Sollwertes dar, wodurch eine schnelle Regelung der Belichtung möglich ist.

Damit der Korrekturwert gewisse Grenzwerte nicht überschreitet, ist in einer Weiterbildung der erfindungsgemäßen Röntgeneinrichtung vorgesehen, daß die Mittel zur Bestimmung des Korrekturwertes eine Begrenzungseinheit zur Begrenzung des Korrekturwertes auf einen vorgegebenen Minimalwert und auf einen Maximalwert 1 aufweisen. Dies soll verhindern, daß die Regelung in bestimmten Grenzsituationen fehlerhaft arbeitet. Der Minimalwert, der beispielsweise bei 0,20 liegen kann, soll dann gelten, wenn die Primärblendenöffnung sehr klein ist und nur ein sehr kleiner Teilbereich, beispielsweise weniger als 20% des Meßfeldes belichtet wird. Der Maximalwert des Korrekturwertes soll 1 betragen und dann gelten, wenn die Primärblendenöffnung so groß ist, daß das gesamte Meßfeld belichtet wird.

Eine weitere Ausgestaltung der Erfindung sieht vor, daß die Mittel zur Bestimmung des Korrekturwertes einen Geber zur Erfassung der Größe der Öffnung der Primärblendenanordnung aufweisen. Dadurch kann dieser Meßwert unmittelbar in den Regelkreis einfließen, beispielsweise in die Bestimmung des Dosis-Sollwertes.

Eine weitere Ausgestaltung der Erfindung sieht vor, daß die Mittel zur Bestimmung des Korrekturwertes einen Geber zur Erfassung der Größe des Abstandes zwischen Röntgenstrahlenquelle und Röntgenbildwandler aufweisen.

Eine bevorzugte Ausgestaltung der Erfindung sieht vor, daß die Röntgeneinrichtung für den Betrieb mit gepulster Durchleuchtung ausgebildet ist und daß die Mittel zur Bestimmung des Korrekturwertes derart ausgebildet sind, daß für jeden Durchleuchtungspuls ein Korrekturwert ermittelt wird. Besonders für den gepulsten Durchleuchtungsbetrieb ist die erfindungsgemäße Röntgeneinrichtung geeignet, da diese eine besonders einfache und schnelle Regelung enthält.

Die Erfindung wird nachfolgend anhand der Zeichnung näher erläutert. Es zeigen:
- Fig. 1: ein Blockschaltbild einer erfindungsgemäßen Röntgeneinrichtung und
- Fig. 2: eine Skizze der Detektorfläche zur Erläuterung der Berechnung des Korrekturwertes.

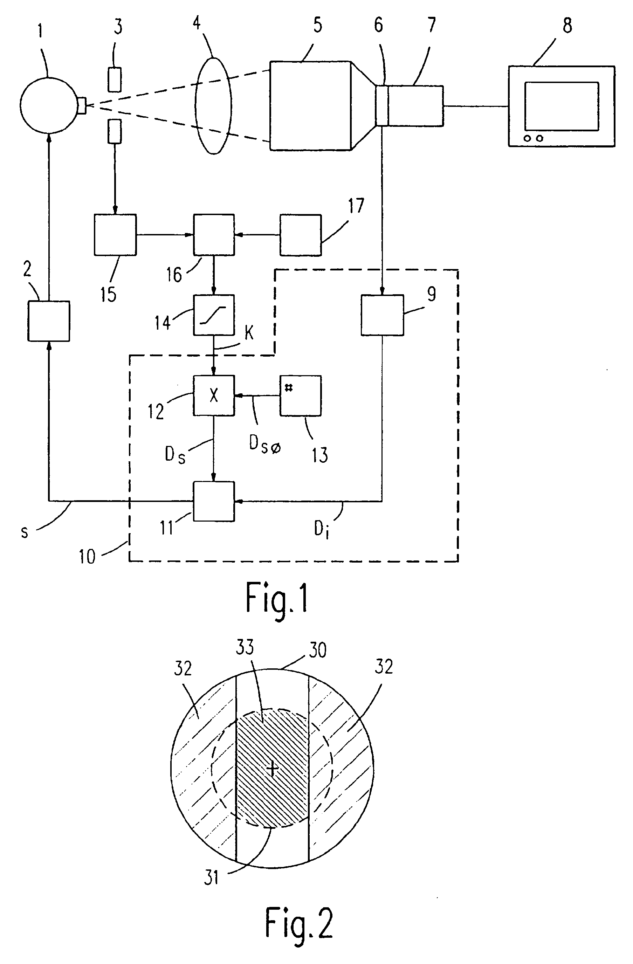

In Fig. 1 ist eine Röntgenröhre 1 gezeigt, die von einem Röntgengenerator 2 gespeist wird. Vor der Strahlenaustrittsöffnung der Röntgenröhre 1 befindet sich eine Primärblende 3, die die austretende Röntgenstrahlung begrenzen und den Öffnungswinkel des Röntgenstrahlenbündels bestimmen kann. Im Strahlengang befindet sich dahinter ein Untersuchungsobjekt 4, wonach ein Röntgenbildverstärker 5 folgt, der mit einer optischen Vorrichtung 6 und einer Fernsehkamera 7 eine Einheit bildet. An der Fernsehkamera 7 ist ein Monitor 8 angeschlossen, auf dem Röntgenbilder dargestellt werden.

Zur automatischen Regelung der Belichtung ist ein Regelkreis 10 vorgesehen, der ein Steuersignal S erzeugt, das dem Röntgengenerator 2 zugeführt wird und mit dem der Röhrenstrom, die Röhrenspannung und/oder die Einschaltdauer der Röntgenstrahlung gesteuert wird. Der Regelkreis 10 weist einen Fotosensor 9 auf, auf den ein Teil der Strahlen des Bildes, das am Ausgangsschirm des Röntgenbildverstärkers 5 erzeugt wird, aus der optischen Vorrichtung 6 beispielsweise mittels eines Strahlteilers oder eines teildurchlässigen Spiegels ausgekoppelt wird. Der Fotosensor 9 wandelt die Helligkeitswerte des ausgekoppelten optischen Bildes in ein Fotosignal. Ein festgelegter Bereich des Fotosensors 9 arbeitet dabei als Meßfeld, in dem indirekt über die Bildhelligkeit die Dosis für die Belichtungsregelung gemessen wird. Im gesamten Meßfeld wird dazu durch Integration die mittlere Bildhelligkeit gemessen und ein Dosis-Istwert Dᵢ als Ausgangswert ermittelt. Dieser Dosis-Istwert Dᵢ wird in der Vergleichseinheit 11 mit einem Dosis-Sollwert D, verglichen, indem beispielsweise diese beiden Werte voneinander subtrahiert werden. Der Dosis-Sollwert Dₛ enthält dabei die Information, wie groß die Belichtung sein muß, damit das Röntgenbild im Bereich des Meßfeldes einen mittleren Schwärzungswert erreicht und das Röntgenbild somit die höchstmögliche Bildqualität aufweist. Aus dem Vergleich in der Vergleichseinheit 11 wird das Steuersignal S erzeugt, das für den Fall, daß der Dosis-Sollwert Dₛ noch nicht erreicht ist, die Information enthält, die Röntgenstrahlendosis weiter zu erhöhen, und anderenfalls, die Röntgenstrahlung abzuschalten.

Erfindungsgemäß wird der Dosis-Sollwert Dₛ im gezeigten Ausführungsbeispiel durch Multiplikation eines Dosis-Standardsollwertes Dₛ₀ mit einem Korrekturwert K in der Multipliziereinheit 12 berechnet. Der Dosis-Standardsollwert Dₛ₀ wird dabei von einer Speichereinheit 13 geliefert, in der Dosis-Standardsollwerte Dₛ₀ für unterschiedliche Bildverstärkerformate, vom Anwender gewählte Dosisniveaus, unterschiedliche Untersuchungsmodi und verschiedene Pulsraten (bei gepulster Durchleuchtung) abgelegt sind.

Bei der Berechnung des Korrekturwertes K spielen mehrere Faktoren eine Rolle. Mittels eines Gebers 15 wird der Wert für die Größe der Öffnung der Primärblendenanordnung 3 und mittels eines Gebers 17 wird der Wert des Abstandes zwischen der Röntgenröhre 1 und dem Röntgenbildverstärker 5 ermittelt.

Diese Werte werden an die Recheneinheit 16 weitergegeben. Die Recheneinheit 16 hat weiterhin die Information gespeichert, wie groß die Fläche des Meßfeldes des Fotosensors 9 ist und welche Position das Meßfeld bezüglich des Ausgangsbildes des Röntgenbildverstärkers 5 einnimmt.

Aus den genannten Werten wird in der Recheneinheit 16 ermittelt, wie groß der Teil der Fläche des Meßfeldes ist, der belichtet und nicht durch die Blenden der Primärblendenanordnung 3 abgeschattet ist. Mit dem belichteten (nicht abgeschatteten) Bereich des Meßfeldes ist dabei gemeint, daß im Bereich des Eingangsschirmes des Röntgenbildverstärkers 5, dem der belichtete Bereich des gewählten Meßfeldes zugeordnet ist, Röntgenstrahlung auftrifft und Bildhelligkeit erzeugt wird. Unter einem unbelichteten (abgeschatteten) Bereich des Meßfeldes ist entsprechend zu verstehen, daß im zugehörigen Bereich des Eingangsschirmes des Röntgenbildverstärkers 5 aufgrund der sehr geringen Öffnung der Primärblendenanordnung 3 keine Röntgenstrahlung auftrifft und demnach auch keine Bildhelligkeit erzeugt wird. Je nach Lage und Größe des Meßfeldes und je nach Größe der Öffnung der Primärblendenanordnung 3 schwankt die Größe des belichteten Bereiches des Meßfeldes.

Anhand von Fig. 2 soll dies nochmals verdeutlicht werden. Mit 30 ist die Oberfläche eines kreisförmigen Fotosensors bezeichnet. Die gestrichelt umrandete Fläche 31 bildet das Meßfeld, das im gezeichten Fall etwa 50% der Gesamtbildfläche des Fotosensors 30 beträgt. Mit 32 sind die Flächen bezeichnet, die aufgrund einer kleinen Öffnung der Primärblendenanordnung 3 durch die Primärblenden abgeschattet sind. In den mit 32 bezeichneten Flächen entsteht also keine Belichtung. Nur der mit 33 bezeichnete (schraffiert dargestellte) Teil der Fläche des Meßfeldes 31 wird belichtet und die in diesem Teil durch Mittelung gemessene Röntgenstrahlendosis soll eine Auswirkung auf die Belichtungsregelung haben.

In der Recheneinheit 16 wird der Korrekturwert K aus dem Verhältnis der nicht abgeschatteten Fläche 33 zur Gesamtfläche 31 des Meßfeldes berechnet, was für den in Figur 2 gezeigten Fall einen Wert für K von etwa 0,75 ergeben würde. Da die Größe des nicht abgeschatteten Fläche 33 des Meßfeldes jedoch nicht direkt gemessen werden kann, werden dazu die der Recheneinheit übergebenen Werte der Größe der Primärblendenöffnung und des Abstandes zwischen Röntgenstrahlenquelle und Röntgenbildwandler zur quasi indirekten Berechnung der nicht abgeschatteten Fläche 33 benutzt.

Mittels einer Begrenzungseinheit 14 wird anschließend der Korrekturwert K auf einen Minimalwert begrenzt, falls sich rechnerisch ein kleinerer Wert für K als der Minimalwert ergeben würde, bzw. wird der Wert von K auf den Wert Eins gesetzt für den Fall, daß das gesamte Meßfeld 31 belichtet ist. Zwischen dem Minimalwert und dem Wert Eins liegende Werte von K bleiben unverändert.

Der Korrekturwert K wird danach in der Multipliziereinheit 12 mit dem Dosis-Standardsollwert Dₛ₀ multipliziert, woraus sich der Dosis-Sollwert Dₛ ergibt. Der Dosis-Standardsollwert Dₛ₀ wird also für den Fall einer teilweisen Abschattung des Meßfeldes 31 herabgesetzt. Dadurch wird verhindert, daß das Steuersignal S fälschlicherweise die Information enthält, die Röntgenstrahlendosis müßte erhöht werden, da der mittlere Schwärzungswert noch nicht erreicht sei, wie dies bei der herkömmlichen Belichtungsregelung der Fall ist, sondern die Röntgenstrahlung wird bereits dann abgeschaltet, wenn im belichteten Bereich des Meßfeldes ein mittlerer Schwärzungswert erreicht ist. Durch die Erfindung wird demnach verhindert, daß die Röntgenbilder überbelichtet und dabei wichtige Bildinformationen vernichtet werden. Außerdem hat die Erfindung den Vorteil, daß der Patient nicht durch eine erhöhte Dosis aufgrund einer nicht optimalen Regelung belastet wird.

Die erfindungsgemäße Röntgeneinrichtung könnte auch derart ausgestaltet sein, daß nicht der Dosis-Standardsollwert verringert, sondern der Dosis-Istwert entsprechend mithilfe des Korrekturwertes erhöht wird bei teilweiser Abschattung des Meßfeldes.

Anstelle eines Strahlteilers und eines Fotosensors zur Auskopplung eines Teils der Strahlung und Messung der Belichtung sind auch andere bekannte Sensoren denkbar wie beispielsweise eine Ionisationskammer zwischen Untersuchungsobjekt 4 und Röntgenbildverstärker 5 oder ein Halbleiter-Strahlenempfänger. Anstelle eines Röntgenbildverstärkers, der optischen Vorrichtung und der Fernsehkamera könnte dann auch eine herkömmliche Filmkassette als Röntgenbildwandler eingesetzt werden.

Bei speziellen Röntgeneinrichtungen sind weiterhin Mittel vorgesehen, die Größe des Meßfeldes zu variieren. Bei derartigen Röntgeneinrichtungen sind dann auch zur Verwirklichung der Erfindung Mittel vorzusehen, die der Recheneinheit (16) die Information über die Größe des aktuellen Meßfeldes übermitteln, beispielsweise ein Meßfeldgeber. Andererseits kann bei Röntgeneinrichtungen, bei denen der Abstand zwischen Röntgenstrahlenquelle und Röntgenbildwandler nicht variiert werden kann, beispielsweise einem C-Bogen-Röntgengerät, der Geber 17 entfallen.

Der Einsatz der Erfindung ist nicht auf spezielle Röntgeneinrichtungen oder Betriebsarten beschränkt. Besonders vorteilhaft ist die Erfindung jedoch bei gepulstem Durchleuchtungsbetrieb einsetzbar, da die erfindungsgemäße Regelung sehr schnell und einfach arbeitet und deshalb für jeden Durchleuchtungspuls ein Steuersignal S ermittelt werden kann.

## Patentansprüche

1. Röntgeneinrichtung mit
- einer Röntgenstrahlenquelle (1) zur Emission eines Röntgenstrahlenbündels,
- einem Röntgenbildwandler (5, 6, 7) zur Detektion von Röntgenstrahlung und Erzeugung eines Röntgenbildes,
- einer zwischen der Röntgenstrahlenquelle (1) und einem Untersuchungsobjekt (4) angeordneten Primärblendenanordnung (3) zur Begrenzung des Röntgenstrahlenbündels,
- einem Regelkreis (10) zur automatischen Belichtungsregelung, welcher einen Detektor (9) mit wenigstens einem die Röntgenstrahlendosisleistung erfassenden Meßfeld (31) aufweist,
**dadurch gekennzeichnet, daß** Mittel (12, 14, 15, 16, 17) zur Bestimmung eines den Regelkreis (10) beeinflussenden Korrekturwertes (K), der von der Größe des belichteten Bereiches (33) des Meßfeldes (31) abhängig ist, und zur Beeinflussung des Regelkreises (10) vorgesehen sind derart, daß auch bei bereichsweise unbelichtetem Meßfeld (31) die Röntgenstrahlendosis nahezu unverändert bleibt.

2. Röntgeneinrichtung nach Anspruch 1,
**dadurch gekennzeichnet, daß** der Regelkreis (10) Mittel (9, 11, 12, 13) zur Bestimmung eines Dosis-Istwertes (Dᵢ), eines Dosis-Sollwertes (Dₛ) und eines Steuersignals (S) durch Vergleich des Dosis-Istwertes (Dᵢ) mit dem Dosis-Sollwert (Dₛ) zur Steuerung der Röntgenstrahlenquelle (1) aufweist.

3. Röntgeneinrichtung nach Anspruch 1 oder 2,
**dadurch gekennzeichnet, daß** die Mittel zur Bestimmung des Korrekturwertes (K) eine Recheneinheit (16) zur Bestimmung des Korrekturwertes (K) durch Bildung des Verhältnisses aus der Fläche des belichteten Bereiches (33) des Meßfeldes (31) zur Gesamtfläche des Meßfeldes (31) und daß der Regelkreis (10) eine Multipliziereinheit (12) zur Bestimmung des Dosis-Sollwertes (Dₛ) durch Multiplikation eines vorgegebenen Dosis-Standardsollwertes (Dₛ₀) mit dem Korrekturwert (K) aufweisen.

4. Röntgeneinrichtung nach einem der vorhergehenden Ansprüche,
**dadurch gekennzeichnet, daß** die Mittel zur Bestimmung des Korrekturwertes (K) eine Begrenzungseinheit (14) zur Begrenzung des Korrekturwertes (K) auf einen vorgegebenen Minimalwert und auf einen Maximalwert 1 aufweisen.

5. Röntgeneinrichtung nach einem der vorhergehenden Ansprüche,
**dadurch gekennzeichnet, daß** die Mittel zur Bestimmung des Korrekturwertes (K) einen Geber (15) zur Erfassung der Größe der Öffnung der Primärblendenanordnung (3) aufweisen.

6. Röntgeneinrichtung nach einem der vorhergehenden Ansprüche,
**dadurch gekennzeichnet, daß** die Mittel zur Bestimmung des Korrekturwertes (K) einen Geber (17) zur Erfassung der Größe des Abstandes zwischen Röntgenstrahlenquelle (1) und Röntgenbildwandler (5, 6, 7) aufweisen.

7. Röntgeneinrichtung nach einem der vorhergehenden Ansprüche,
**dadurch gekennzeichnet, daß** die Röntgeneinrichtung für den Betrieb mit gepulster Durchleuchtung ausgebildet ist und daß die Mittel zur Bestimmung des Korrekturwertes (K) derart ausgebildet sind, daß für jeden Durchleuchtungspuls ein Korrekturwert (K) ermittelt wird.

## Claims

1. An X-ray device, including
- an X-ray source (1) for emitting an X-ray beam,
- an X-ray image converter (5, 6, 7) for detecting X-rays and for forming an X-ray image,
- a primary diaphragm device (3) which, in order to limit the X-ray beam, is arranged between the X-ray source (1) and an object to be examined,
- a control circuit (10) for automatic exposure control, including a detector (9) with at least one measuring field (31) for measuring the X-ray dose rate,
**characterized in that** there are provided means (12, 14, 15, 16, 17) for determining a correction value (K) which influences the control circuit (10) and is dependent on the dimensions of the exposed area (33) of the measuring field (31), and for influencing the control circuit (10) in such a manner that the X-ray dose remains substantially the same even when parts of the measuring field (31) are not exposed.

2. An X-ray device as claimed in claim 1,
**characterized in that** the control circuit (10) includes means (9, 11, 12, 13) for determining an actual dose value (Dᵢ), a reference dose value (Dₛ) and a control signal (S) by comparison of the actual dose value (Dᵢ) with the reference dose value (Dₛ) for controlling the X-ray source (1).

3. An X-ray device as claimed in claim 1 or 2,
**characterized in that** the means for determining the correction value (K) include an arithmetic unit (16) for determining the correction value (K) by forming the ratio of the surface area of the exposed area (33) of the measuring field (31) to the overall surface area of the measuring field (31), and that the control circuit (10) includes a multiplier unit (12) for determining the reference dose value (Dₛ) by multiplication of a given dose standard reference value (Dₛ₀) by the correction value (K).

4. An X-ray device as claimed in one of the preceding claims,
**characterized in that** the means for determining the correction value (K) include a limiter unit (14) for limiting the correction value (K) to a predetermined minimum value and to a maximum value 1.

5. An X-ray device as claimed in one of the preceding claims,
**characterized in that** the means for determining the correction value (K) include a sensor (15) for sensing the value of the aperture of the primary diaphragm device (3).

6. An X-ray device as claimed in one of the preceding claims,
**characterized in that** the means for determining the correction value (K) include a sensor (17) for sensing the distance between the X-ray source (1) and the X-ray image converter (5, 6, 7).

7. An X-ray device as claimed in one of the preceding claims,
**characterized in that** the X-ray device is constructed for pulsed fluoroscopy, and that the means for determining the correction value (K) are constructed so that a correction value (K) is determined for each fluoroscopy pulse.

## Revendications

1. Appareil à rayons X avec
- une source de rayons X (1) pour l'émission d'un faisceau de rayons X,
- un convertisseur d'image radiographique (5, 6, 7) pour la détection de rayons X et la production d'une image radiographique,
- un diaphragme primaire (3) disposé entre la source de rayons X (1) et un objet d'examen (4) en vue de délimiter le faisceau de rayons X,
- un circuit de réglage (10) pour le réglage automatique de l'exposition qui comprend un capteur (9) avec au moins un champ de mesure (31) enregistrant la puissance de la dose de rayons X,
**caractérisé en ce que** des moyens (12, 14, 15, 16, 17) sont utilisés pour déterminer une valeur de correction (K) influençant le circuit de réglage (10) qui dépend de la taille de la zone exposée (33) du champ de mesure (31) et pour influencer le circuit de réglage (10) de telle sorte que la dose de rayons X reste pratiquement inchangée, même en cas de champ de mesure (31) non exposé par endroits.

2. Appareil à rayons X selon la revendication 1,
**caractérisé en ce que** le circuit de réglage (10) présente des moyens (9, 11, 12, 13) pour la détermination d'une valeur réelle de la dose (Dᵢ), d'une valeur de consigne de la dose (Dₛ) et d'un signal de commande (S) par comparaison de la valeur réelle de la dose (Dᵢ) avec la valeur de consigne de la dose (Dₛ) pour la commande de la source de rayons X (1).

3. Appareil à rayons X selon l'une des revendications 1 ou 2,
**caractérisé en ce que** les moyens de détermination de la valeur de correction (K) présentent une unité de calcul (16) en vue de la détermination de la valeur de correction (K) par formation du rapport entre la surface de la zone exposée (33) du champ de mesure (31) et la surface totale du champ de mesure (31) et que le circuit de réglage (10) présente une unité du multiplicateur (12) pour la détermination de la valeur de consigne de la dose (Dₛ) par multiplication d'une valeur de consigne standard de la dose (D_{sO}) préalablement déterminée avec la valeur de correction (K).

4. Appareil à rayons X selon l'une des revendications précédentes, **caractérisé en ce que** les moyens de détermination de la valeur de correction (K) présentent une unité de délimitation (14) pour la délimitation de la valeur de correction (K) à une valeur minimale préalablement déterminée et à une valeur maximale 1.

5. Appareil à rayons X selon l'une des revendications précédentes, **caractérisé en ce que** les moyens de détermination de la valeur de correction (K) présentent un capteur (15) en vue de l'enregistrement de la taille de l'ouverture du diaphragme primaire (3).

6. Appareil à rayons X selon l'une des revendications précédentes **caractérisé en ce que** les moyens de détermination de la valeur de correction (K) présentent un capteur (17) en vue de l'enregistrement de la taille de l'intervalle entre la source de rayons X (1) et les convertisseurs d'images radiographiques (5, 6, 7).

7. Appareil à rayons X selon l'une des revendications précédentes,
**caractérisé en ce que** l'appareil à rayons X est conçu pour le fonctionnement avec une radioscopie pulsée et que les moyens de détermination de la valeur de correction (K) sont conçus de telle sorte qu'une valeur de correction (K) soit déterminée pour chaque impulsion de radioscopie.
